**Europäisches Patentamt**

**European Patent Office**

**Office européen des brevets**

(11) Veröffentlichungsnummer: **0 229 990 B1**

## EUROPÄISCHE PATENTSCHRIFT

(45) Veröffentlichungstag der Patentschrift: **22.04.92**

(21) Anmeldenummer: **86117359.9**

(22) Anmeldetag: **12.12.86**

(51) Int. Cl.⁵: **C12P 19/06**

(54) **Verfahren zur Herstellung von exozellulären Biopolymeren mit Verdickungswirkung für wässrige Medien.**

(30) Priorität: **20.12.85 DE 3545246**

(43) Veröffentlichungstag der Anmeldung:
**29.07.87 Patentblatt 87/31**

(45) Bekanntmachung des Hinweises auf die
Patenterteilung:
**22.04.92 Patentblatt 92/17**

(84) Benannte Vertragsstaaten:
**AT BE CH DE FR GB IT LI NL SE**

(56) Entgegenhaltungen:
**EP-A- 0 074 775**
**EP-A- 0 187 092**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Viehweg, Holger, Dr.
Weissenstein 83
W-4018 Langenfeld(DE)**

(74) Vertreter: **von Kreisler, Alek, Dipl.-Chem. et al
Patentanwälte Von Kreisler-Selting-Werner,
Deichmannhaus am Hauptbahnhof
W-5000 Köln 1(DE)**

**Beschreibung**

Gegenstand der europäischen Patentanmeldung 0 058 364 ist ein Verfahren zur Herstellung von Xanthomonas-Biopolymeren durch aerobes Züchten von Mikroorganismen der Gattung Xanthomonas in einem wässrigen Nährmedium, das dadurch gekennzeichnet ist, daß man die Mikroorganismen in einer unter Fermentationsbedingungen stabilen Wasser-in-Öl-Emulsion (W/O-Emulsion) züchtet. Man kann dabei so vorgehen, daß man den Mikroorganismus zunächst im ölfreien, wässrigen Nährmedium anzüchtet, dann unter Zusatz der Ölphase eine W/O-Emulsion ausbildet und unter Aufrechterhaltung der W/O-Emulsion die Fermentation weiterführt. Bevorzugt wird in Gegenwart von W/O-Emulgatoren gearbeitet, wobei zweckmäßigerweise zunächst der W/O-Emulgator in der Ölphase gelöst und diese Lösung dann mit der wässrigen Phase vermischt wird.

Gegenstand der europäischen Patentanmeldung 0 098 473 ist eine Erweiterung dieses Verfahrens dahingehend, daß man in solchen W/O-Emulsionen allgemein exozelluläre Biopolymere mit Verdickungswirkung für das wässrige Nährmedium bildende Mikroorganismenstämme züchtet, die nicht der Gattung Xanthomonas angehören. Genannt sind beispielsweise gram-positive oder gram-negative Bakterienstämme, Hefen, Pilze oder Algen. Eine entsprechende Lehre wird in der US-PS 4,352,882 gegeben, wobei auch dort der Züchtung von Xanthomonasstämmen besondere Bedeutung gegeben ist.

Durch den Einsatz solcher W/O-Emulsionen bei der aeroben Züchtung von beispielsweise Xanthan bildenden Xanthomonasstämmen können in mehrfacher Weise wichtige Vorteile eingestellt werden. Ausgangspunkt der Untersuchungen war, durch die Wahl der W/O-Emulsionstechnik die Schwierigkeiten zu mildern beziehungsweise zu beseitigen, die in der wässrigen Fermentationsphase für den Verfahrensablauf durch die zunehmende Verdickung eintreten, die durch das als Fermentationsprodukt anfallende Polysaccharid ausgelöst wird. Beim Arbeiten mit rein wässrigen Nährmedien sind Ausbeuten von 2 bis 3 Gewichtsprozent Xanthan (Trockensubstanz) bereits als gutes Ergebnis zu werten, das nur unter Einsatz stark Energie aufwendiger Spezialmaßnahmen erhalten werden kann. Beim Übergang auf die Züchtung der Polysaccharide bildenden Mikroorganismenstämme in der wässrigen dispersen Phase von W/O-Emulsionen gelingt in erster Linie eine Senkung der Viskosität der Flüssigphase im Fermenter unter Fermentationsbedingungen, da die Viskosität des Gesamtsystems in erster Linie durch die homogene Ölphase bestimmt wird. Es ist auch schon festgestellt worden, daß beim Übergang auf solche W/O-Emulsionssysteme verbesserte Xanthanausbeuten in der dispersen wässrigen Phase im Vergleich zum Arbeiten mit einem rein wässrigen Züchtungsmedium erhalten werden können.

Die EP-A-0 187 092 betrifft ein Verfahren zur Herstellung von Polysacchariden durch Fermentation von Kohlehydraten mit Hilfe von Mikroorganismen Die Fermentation wird in einer W/O-Emulsion durchgeführt.

Die EP-A-0 074 775 betrifft ein Verfahren zur Herstellung von Xanthan-Gummi, das durch Dispersion eines wäßrigen Xanthamonas-Kulturmediums in einem wasserunlöslichen Öl, beispielsweise einem Kohlenwasserstoff oder einem Speiseöl, durchgeführt wird. Derartige Dispersionen weisen eine geringere Viskosität auf und können mit einer geringeren Energie zu einem Polymer fermentiert werden.

Die europäische Patentanmeldung 0 135 123 betrifft ein Verfahren zur Verringerung der Viskosität von Fermentationsbrühen durch Zusatz eines halogenierten Kohlenwasserstoffs und Ausbildung entsprechender W/O-Emulsionen. Dabei werden als halogenierte Kohlenwasserstoffphasen ungiftige, unter den Fermentationsbedingungen flüssige, hoch halogenierte Aliphaten eingesetzt, die insbesondere perhalogniert sind. Auf den Einsatz von Emulgatoren wird verzichtet. Diese Lehre geht von Erkenntnissen aus, wonach bei den zuvor geschilderten Verfahren zur Xanthanbildung in W/O-Emulsionen die Viskosität des Gesamtmediums gegebenenfalls nicht nur nicht verringert, sondern sogar erhöht wird. Diese Gefahr soll insbesondere beim Einsatz von Emulgatoren bestehen.

Weitere Untersuchungen haben diese Schwierigkeiten nicht bestätigen können. Es taucht im Gegenteil beim Versuch der Optimierung der Verfahrensbedingungen eine entgegengesetzte Problematik auf: Zwei für die wirtschaftliche Durchführung eines solchen Mikroorganismen-Züchtungsverfahrens in W/O-Emulsionen wesentliche Verfahrensparameter ergänzen sich bei ihrer Optimierung dergestalt in unerwünschter Weise, daß die W/O-Emulsionen zum Brechen und damit zur Phasentrennung neigen, wobei es schwierig oder unmöglich wird, durch einfache Mittel - insbesondere durch Rühren - den gewünschten Zustand der fein dispersen W/O-Emulsion wieder herzustellen. Die beiden Verfahrensparameter, um die es sich hier handelt, sind zum einen die Menge der Ölphase und zum anderen die zunehmende Anreicherung der Feststoffphase mit dem extracellulären Polysaccharid, d.h.die Ausbeute in der dispersen wässrigen Phase.

Begreiflicherweise ist es wünschenswert, mit möglichst geringen Mengen an Ölphase zu arbeiten, um die Kosten des Gesamtverfahrens aus dieser Sicht heraus so niedrig wie möglich zu halten. Zum anderen ist es selbstverständlich, daß möglichst hohe Ausbeuten an extrazellulärem Polysaccharid mit Verdickungswirkung in der dispersen wässrigen Phase angestrebt werden. Es zeigt sich nun, daß die erwähnte Instabilität der W/O-Emulsionen insbesondere dann zu befürchten ist, wenn die Menge der Ölphase so weit wie möglich gesenkt und gleichzeitig die Ausbeute an extrazellulärem Polysaccharid in der dispersen wässrigen Phase so hoch wie möglich getrieben wird. In dieser für die Wirtschaftlichkeit des Verfahrens besonders günstigen Kombination der beiden Parameter ist mit ernsten Verfahrensstörungen durch unerwünschte und irreversible Phasentrennung zu rechnen, so daß damit ein vorzeitiger Abbruch des Züchtungsverfahrens unvermeidlich wird.

Aufgabe der vorliegenden Erfindung war es, diesen Schwierigkeiten zu begegnen. Die technische Lösung der erfindungsgemäßen Aufgabe liegt in der Feststellung, daß eine besonders ausgewählte Emulgatorklasse aus der Vielzahl an sich bekannter W/O-Emulgatoren geeignet ist, hier Abhilfe zu schaffen und damit gleichzeitig die Möglichkeit gibt, die Menge der Ölphase derart zu reduzieren, daß - gewichts- und volumenmäßig gesehen - die wässrige Phase im Gesamtansatz weit überwiegt und gleichwohl stabile W/O-Emulsionen selbst dann noch vorliegen, wenn die Feststoffausbeuten in der dispersen wässrigen Phase zu optimalen Werten hochgetrieben werden.

Gegenstand der Erfindung ist dementsprechend ein Verfahren zur Herstellung von exozellulären Biopolymeren mit Verdickungswirkung für wässrige Medien durch aerobes Züchten von die Biopolymeren bildenden Mikroorganismenstämmen in einem wässrigen Nährmedium, das als disperse wässrige Phase in einer unter Fermentationsbedingungen stabilen W/O-Emulsion vorliegt unter Mitverwendung von W/O-Emulgatoren zur Emulsionsbildung und -stabilisierung, wobei das Verfahren dadurch gekennzeichnet ist, daß mit einer Ölphase in Mengen unterhalb 50 Volumprozent - bezogen auf Gesamtansatz - gearbeitet wird und dabei Fettsäuredialkanolamide als W/O-Emulgatoren eingesetzt werden. Die bevorzugten Fettsäuredialkanolamide sind entsprechende Diethanolamide, deren Fettsäurereste auch ein- oder mehrfach olefinisch ungesättigt sein können. Der Begriff der Fettsäuren erfaßt dabei in bekannter Weise die Monocarbonsäuren, insbesondere natürlichen Ursprungs, des bevorzugten C-Zahlbereichs von 12 bis 20, insbesondere 14 bis 18. Bekannte einfach oder mehrfach olefinisch ungesättigte natürliche Fettsäuren sind Ölsäure, Linolsäure und Linolensäure.

Es hat sich gezeigt, daß schon sehr geringe Mengen der erfindungsgemäß ausgewählten Emulgatoren befähigt sind, stabile W/O-Emulsionen selbst dann zu gewährleisten, wenn mit einem Minimum an Ölphase und gleichzeitig maximalen Feststoffausbeuten in der wässrigen Phase gearbeitet wird. Bevorzugt liegt die eingesetzte Emulgatormenge im Bereich von etwa 0,5 bis 2 Gewichtsprozent, insbesondere im Bereich von etwa 0,7 bis 1,2 Gewichtsprozent - jeweils bezogen auf den Gesamtansatz.

Die Ölphase wird in der bevorzugten Ausführungsform in Mengen unter 40 Volumprozent - bezogen auf das Gemisch von Wasser und Öl - verwendet, wobei Mengen der Ölphase im Bereich von etwa 30 bis 40 Volumprozent geeignet sein können. 35 Volumprozent entsprechen dabei etwa 25 Gewichtsprozent - jeweils wieder bezogen auf den Gesamtansatz. Mit diesen geringen Ölmengen können stabile vergleichsweise leicht bewegliche W/O-Emulsionen selbst dann erhalten werden, wenn man die erfindungsgemäß ausgewählten Emulgatoren einsetzt und die aerobe Züchtung der exozellulären Polysaccharide auf Feststoffausbeuten über 5 Gewichtsprozent und insbesondere über 7 Gewichtsprozent - auch hier jeweils bezogen auf das Gesamtgemisch - treibt. Das erfindungsgemäße Handeln ermöglicht damit, die Vorteile der Emulsionstechnik - die unter anderem in der erhöhten Beweglichkeit des Reaktionsgemisches liegen - mit dem Vorteil besonderer Wirtschaftlichkeit zu kombinieren.

Zu den Einzelheiten der Verfahrensführung gelten die Angaben des eingangs zitierten Stand der Technik. Besonders geeignete Ölphasen sind dementsprechend Isoparaffinkohlenwasserstoffe, die unter Verhafrenstemperatur (30 $\mp$ 5 °C) flüssig sind. Das unter dem Zeichen Isopar® M vertriebene Isoparaffingemisch kann unter den erfindungsgemäßen Bedingungen mit Vorteil verwendet werden. Für die Herstellung von verdickenden Polysacchariden mit Nahrungsmittelqualität kann die Verwendung von physiologisch unbedenklichen Ölphasen, insbesondere von pflanzlichen Ölphasen besonders geeignet sein. Zu nennen sind hier die bekannten flüssigen Triglyceride, die auch als Speiseöle bezeichnet werden.

Ein besonderer Vorteil der erfindungsgemäßen Lehre liegt darin, daß nicht nur unter den angestrebten Extrembedingungen hinreichend stabile W/O-Emulsionen erhalten werden, die unter wirtschaftlich besonders günstigen Bedingungen die Züchtung der Polysaccharid bildenden Organismenstämme gestatten, sondern daß darüber hinaus trotz der angestrebten Emulsionsstabilität bei Verfahrensende eine einfache Trennung zwischen Ölphase und mit Fermentationsprodukt beladener wässriger Phase möglich ist.

Im übrigen gelten für die Durchführung des erfindungsgemäßen Verfahrens die aus dem eingangs zitierten Stand der Technik bekannten Maßnahmen.

Das wässrige Fermentationsmedium kann unter den in der Literatur für den jeweiligen Mikroorganismus beschriebenen ausgewählt werden. Geeignete wässrige Fermentationsmedien sind beispielsweise beschrieben bei: J. R. Norris, D. W. Ribbons (Herausg.): Methods in Microbiology, Vol. 3 A, Academic Press London (1970), oder bei R. L. Whistler B. N. Bemiller (Herausg.): Industrial Gums, Polysaccharids and Derivativs (1973) im gleichen Verlag.

Typische wässrige Nährlösungen enthalten beispielsweise eine Quelle für organischen Stickstoff wie Maisquellwasser und/oder Sojamehl, Phosphatsalze wie Dialkalihydrogenphosphat und/oder Diammoniumhydrogenphosphat neben geeigneten Spurenelementen, insbesondere Magnesium und gegebenenfalls Mangan, Molybdän, Eisen und Calcium bei einem pH-Wert oberhalb 6, vorzugsweise oberhalb von 6,5 bis etwa 7. Die Nährlösung enthält zusätzlich ein geeignetes Kohlenhydrat zweckmäßigerweise in der wässrigen Phase gelöst. Geeignete Kohlenhydrate sind zum Beispiel Glucose, Saccharose, Maltose, Fructose, Lactose, bearbeitete, invertierte Zuckerrüben-Molassen, Invertzucker, filtrierte verdünnte Qualitätsstärke oder Gemische dieser Kohlenhydrate. Glucose ist eine bevorzugte Quelle des assimilierbaren Kohlenstoffs. Die Konzentration der assimilierbaren Kohlenhydratverbindung liegt üblicherweise im Bereich von 0,5 bis 5 Gewichtsprozent, bezogen auf die wässrige Phase. Die Verwendung höherer Konzentrationen an assimilierbaren Kohlehydratverbindungen kann zur Ansammlung toxischer Nebenprodukte und damit zu einer Hemmung des Wachstums der Mikroorganismen führen. Auch können niedermolekulare, für viele Verwendungszwecke nicht geeignete, Stoffwechselprodukte entstehen. In manchen Fällen wird sogar eine vorzeitige Beendigung der Fermentation ausgelöst.

Möglich ist jedoch die Zugabe der assimilierbaren Kohlenhydratverbindung absatzweise oder kontinuierlich während des Fermentationsverlaufs, so daß letztlich beträchtlich höhere Glucosekonzentrationen umgesetzt werden können. Eine besondere Ausführungsform der Erfindung besteht darin, n-Paraffine mit mehr als 10 C-Atomen als Ölphase zu verwenden und gleichzeitig Mikroorganismen zu züchten, welche diese als Quelle assimilierbaren Kohlenstoffs verwerten können. Derartige Mikroorganismen sind aus den Gattungen Corynebacterium, Brevibacterium und Mycobacterium bekannt, zum Beispiel C. viscosum oder M. Lacticolum.

In allen Fällen liegt die Inkubationstemperatur zweckmäßigerweise im Bereich von etwa 30 °C, beispielsweise bei 30 ± 10 °C. Die Fermentation kann bis zu einem Zeitraum von etwa 100 Stunden oder auch länger durchgeführt werden.

Geeignete Xanthomonasorganismen, die exozelluläre Heteropolysaccharide bilden können, leiten sich beispielsweise aus den folgenden Xanthomonas-Species ab: Xanthomonas campestris, Xanthomonas phaseoli, Xanthomonas malvacearum, Xanthomonas translucens, Xanthomonas carotae, Xanthomonas hederae, Xanthomonas papavericola, Xanthomonas begoniae, Xanthomonas incanae, Xanthomonas vasculorum und Xanthomonas vesicatoria. Geeignet sind insbesondere Stämme von Xanthomonas campestris aber auch solche von Xanthomonas fragaria, Xanthomonas gummisudans, Xanthomonas manihotis und Xanthomonas vasculorum. Weitere geeignete Mikroorganismenstämme, die exozelluläre Heteropolysaccharide bilden können, nicht aber der Gattung Xanthomonas zuzuordnen sind, sind im einzelnen in der eingangs zitierten europäischen Patentanmeldung 0 098 473 aufgeführt.

Zur Ausbildung der W/O-Emulsion werden die angezüchtete Nährlösung und die Ölphase - in der zweckmäßigerweise zuvor der gegebenenfalls mitverwendete Emulgator gelöst worden ist - miteinander vermischt und das Gemisch hinreichend intensiv mechanisch bewegt beziehungsweise durchgearbeitet. In der bevorzugten Ausführungsform des erfindungsgemäßen Verfahrens erfolgt die Züchtung auch innerhalb der W/O-Emulsion in Fermentern, die mit intensiv arbeitenden Mischelementen ausgerüstet sind. Die intensive Durchmischungswirkung solcher Rührelemente kann erfindungsgemäß dazu ausgenutzt werden, im Fermenter den Zustand der W/O-Emulsion aufrechtzuerhalten und sicherzustellen, und zwar selbst in solchen Fällen, in denen bei Ruhigstellung des Fermenterinhalts eine relativ einfache Phasenentmischung eintritt.

Der bewegte Fermenterinhalt wird in konventioneller Weise mit Sauerstoff beziehungsweise mit einem sauerstoffhaltigen Gas, insbesondere Luft, begast. Der Sauerstofftransfer erfolgt über die Ölphase an jedes einzelne in disperser Phase emulgierte Teilchen der wäßrigen, Mikroorganismen enthaltenden Nährlösung und ist dementsprechend auch in späten Stadien des Verfahrens weitaus weniger gestört als bei der bisher üblichen Verfahrenstechnik.

Erforderlichenfalls können wachstumsfördernde Bestandteile der Nährlösung absatzweise oder kontinuierlich nachgeliefert werden. So ist es etwa möglich, die assimilierbaren Kohlenstoff enthaltenden Verbindungen wie Glucose nach und nach dem Reaktionsgemisch zuzusetzen.

Eine gleichmäßige Verteilung kann dadurch sichergestellt werden, daß diese Nährstoffe dem intensiv durchgearbeiteten Fermenter unmittelbar zugegeben werden. Entsprechendes gilt für andere Bestandteile der wässrigen Nährlösung, beispielsweise für Spurenelemente oder auch für die Zugabe von sonstigen benötigten Reaktanten, beispielsweise Basen, zur Regulierung des pH-Wertes der dispersen wässrigen Phase.

Die Züchtung wird dann solange fortgeführt, bis die gewünschte Ausbeute eingestellt ist beziehungsweise die Biopolymerbildung absinkt oder gar zum Stillstand kommt. Im absatzweisen Verfahren wird dann

EP 0 229 990 B1

- je nach dem beabsichtigten Verwendungszweck des Produktes - in der Regel eine Trennung zwischen Ölphase und Biopolymer haltiger disperser Phase erfolgen, woraufhin das Polysaccharid in konventioneller Weise aus der wässrigen Phase abgetrennt und gereinigt werden kann. Zum Brechen der W/O-Emulsionen können die in der chemischen Verfahrenspraxis üblichen Maßnahmen eingesetzt werden, vergleiche hierzu beispielsweise Ullmann Enzyklopädie der technischen Chemie 1975, Band IV, Seite 453 und ff. sowie Houben-Weyl, Methoden der Organischen Chemie 1958, Band I/1, Seiten 219/220. Die Emulsionstrennung kann dementsprechend durch Zugabe emulsionsbrechender Substanzen, gegebenenfalls durch Einwirkung mechanischer Kräfte, wie Schütteln, Schlagen oder Druckeinwirkung, durch Temperaturerhöhung, durch Verdünnen oder Eindampfen der äußeren Phase und andere bekannte Maßnahmen erfolgen. Insbesondere die Zerstörung von Emulsionen durch Zusatz geeigneter chemischer Stoffe, der sogenannten Desmulgatoren, ist in der Verfahrenstechnik bekannt. Der Handel hat eine Vielzahl von Emulsionsspaltern entwickelt, vergleiche hierzu beispielsweise Houben-Weyl aaO. Übliche Reinigungsschritte, beispielsweise das Auswaschen der Biopolymer haltigen wässrigen Phase mit geeigneten Lösungsmitteln, können sich anschließen.

In einer besonderen Ausführungsform des erfindungsgemäßen Verfahrens kann ein Teilstrom des Fermenterinhaltes absatzweise oder kontinuierlich abgezweigt, aufgearbeitet und gewünschtenfalls wenigstens anteilsweise wieder zurückgegeben werden. Über den abgezweigten Teilstrom kann beispielsweise die Zugabe von Reaktionshilfsmitteln oder aber auch die Zugabe frischer Mikroorganismenanteile erfolgen. Gleichzeitig kann über einen solchen abgezweigten Teilstrom eine absatzweise oder kontinuierliche Ausschleusung von Verfahrensprodukt aus dem Fermenter erfolgen. Für die Erfindung eröffnet sich hiermit die Möglichkeit der kontinuierlichen Verfahrensführung, wobei insbesondere durch regelmäßige Kontrolle der das Biopolymer enthaltenden wässrigen Phase die Steuerung des Verfahrensablaufs in gewünschter Richtung möglich ist. Gegenüber dem in der Praxis bisher üblichen Verfahrenstyp des absatzweise arbeitenden einstufigen Verfahrens erschließen sich hier neue technisch fortschrittliche Möglichkeiten.

Die Isolierung des Polysaccharids aus der wässrigen Phase erfolgt in an sich bekannter Weise, beispielsweise durch Fällung und Trocknung. Zunächst kann die wässrige Phase hinreichend - zum Beispiel auf Temperaturen über 100 °C - erhitzt und sofort wieder abgekühlt werden, um die vorhandenen Mikroorganismen abzutöten und gegebenenfalls die Viskosität des Biopolymeren zu verbessern. Das Biopolymer wird dann durch Fällung, beispielsweise mit Alkoholen, anschließendes Filtrieren und Trocknen gewonnen. Waschstufen zur Reinigung des Produkts können in an sich bekannter Weise eingeschlossen sein.

Beispiele

Beispiel 1:

Xanthomonas campestris NRRL-B-1459-A wurde im 15 l-Fermenter (10 l Füllvolumen) bei 28 °C aerob gezüchtet:
Der Fermenter wurde mit einer 24 Std. alten Vorkultur beimpft.

5

Nährmedium der Hauptkultur:

| | | | | |
|---|---|---|---|---|
| Maisquellwasser | 10 | g/l | Drehzahl 2200 $min^{-1}$ | |
| Sojamehl | 6 | g/l | Luft | 1 VVM |
| $Na_2HPO_4 \times 12\ H_2O$ | 0,9 | g/l | | |
| $(NH_4)_2HPO_4$ | 0,8 | g/l | | |
| $MGSO_4 \times 7\ H_2O$ | 0,2 | g/l | | |
| Öl: Isopar® M * | 250 | g/l | | |
| Emulgator: | | | | |
| Linolsäurediäthanolamid | 8 | g/l | | |

\*                                                     Isoparaffingemisch des Siedebereiches 200 bis 250 $^O$C.

Nach dem Ansetzen der Kulturlösung wurde der Reaktor sterilisiert und beimpft. Die Glukose wurde während der Fermentation ständig zudosiert, so daß die Konzentration ständig bei etwa 10 g/l lag. Der pH-Wert wurde mit Kalilauge konstant bei pH 7,0 gehalten. Nach 30 Stunden Fermentationsdauer wurde der Rührer kurz abgestellt und das extern sterilisierte Öl mit dem Emulgator dazugegeben. Nach der Ölzugabe wurde die Fermentation wie zuvor weitergeführt.

Nach einer Kulturdauer von etwa 160 Stunden betrug die Xanthankonzentration 61 g/l.

Beispiel 2:

In einem zweiten Versuch wurde mit dem Stamm ATCC 31 602 in gleicher Weise gearbeitet. Als Emulgator wurde in diesem Versuch jedoch das Handelsprodukt "Comperlan® VOD" eingesetzt. Es handelt sich hierbei um ein Fettsäurediäthanolamid auf Basis pflanzlicher Öle.

Nach dem Abbruch der Fermentation, in der 110. Stunde, wurde die Xanthankonzentration mit 65 g/l bestimmt.

Vergleichsbeispiel

Eine Vergleichsfermentation mit einem anderen Emulgator wurde unter sonst gleichen Bedingungen durchgeführt. Anstelle von Linolsäurediäthanolamid wurde in diesem Fall der von der Anmelderin unter dem Handelsnamen "Dehymuls® F" vertriebene Emulgator eingesetzt, der ein Gemisch aus höhermolekularen Fettsäureestern darstellt.

Nach Erstellung der Emulsion mußte die Fermentation abgebrochen werden, da die Emulsion nicht stabil blieb und damit keine Xanthanbildung mehr möglich war. Die Xanthankonzentration betrug nach Abbruch 20 g/l.

**Patentansprüche**

1.     Verfahren zur Herstellung von exozellulären Biopolymeren mit Verdickungswirkung für wässrige Medien durch aerobes Züchten von die Biopolymeren bildenden Mikroorganismenstämmen in einem wässrigen Nährmedium, das als disperse wässrige Phase in einer unter Fermentationsbedingungen stabilen Wasser/Öl-Emulsion (W/O-Emulsion) vorliegt, unter Mitverwendung von W/O-Emulgatoren zur Emulsionsbildung und -stabilisierung, dadurch gekennzeichnet, daß die Ölphase in Mengen unter 50 Volumenprozent - bezogen auf den Gesamtansatz - zum Einsatz kommt und dabei mit Fettsäuredialkanolamiden als W/O-Emulgatoren gearbeitet wird.

2.     Verfahren nach Anspruch 1, dadurch gekennzeichnet, daß Fettsäurediethanolamide eingesetzt werden, deren Fettsäurereste auch ein- oder mehrfach olefinisch ungesättigt sein können.

**3.** Verfahren nach Ansprüchen 1 und 2, dadurch gekennzeichnet, daß der Emulgator in Mengen von 0,5 bis 2 Gewichtsprozent, vorzugsweise von 0,7 bis 1,2 Gewichtsprozent - jeweils bezogen auf Gesamtansatz - eingesetzt wird.

**4.** Verfahren nach Ansprüchen 1 bis 3, dadurch gekennzeichnet, daß mit bei Verfahrenstemperatur (30 ± 5 °C) flüssigen Isoparaffin - Kohlenwasserstoffen beziehungsweise flüssigen Triglyceriden, insbesondere Speiseölen, als homogene Phase gearbeitet wird.

**5.** Verfahren nach Ansprüchen 1 bis 4, dadurch gekennzeichnet, daß die Ölphase in einer Menge von 30 bis 40 Volumenprozent - bezogen auf Gesamtansatz - eingesetzt wird.

**6.** Verfahren nach Ansprüchen 1 bis 5, dadurch gekennzeichnet, daß Biopolymerausbeuten von wenigstens 5 Gewichtsprozent - bezogen auf Gesamtansatz - in der dispersen wässrigen Phase eingestellt werden, bevor das Verfahrensprodukt der Biopolymerabtrennung zugeführt wird.

**7.** Verfahren nach Ansprüchen 1 bis 6, dadurch gekennzeichnet, daß es zur Herstellung von Polysaccharid-Biopolymeren, insbesondere der Gattung Xanthomonas eingesetzt wird.

**Claims**

**1.** A process for the preparation of exocellular biopolymers having a thickening effect for aqueous media by aerobic culture of microorganism strains forming the biopolymers in an aqueous nutrient medium present as a disperse aqueous phase in a water/oil emulsion (W/O emulsion) stable under fermentation conditions using W/O emulsifiers for emulsion formation and stabilization, characterized in that the oil phase is used in quantities below 50% by volume, based on the mixture as a whole, and in that fatty acid dialkanolamides are used as the W/O emulsifiers.

**2.** The process according to claim 1, characterized in that fatty acid diethanolamides are used, the fatty acid residues of which may also be mono- or poly-olefinically unsaturated.

**3.** The process according to claims 1 and 2, characterized in that the emulsifier is used in amounts of from 0.5 to 2% by weight, and preferably of from 0.7 to 1.2% by weight, each based on the mixture as a whole.

**4.** The process according to claims 1 to 3, characterized in that isoparaffin hydrocarbons liquid at the process temperature (30 ± 5 °C) or liquid triglycerides, and particularly edible oils, are used as the homogeneous phase.

**5.** The process according to claims 1 to 4, characterized in that the oil phase is used in a quantity of from 30 to 40% by volume, based on the mixture as a whole.

**6.** The process according to claims 1 to 5, characterized in that biopolymer yields of at least 5% by weight, based on the mixture as a whole, are achieved in the disperse aqueous phase, before the product of the process is delivered to the biopolymer separation stage.

**7.** The process according to claims 1 to 6, characterized in that it is used for the preparation of polysaccharide biopolymer, particularly of the genus Xanthomonas.

**Revendications**

**1.** Procédé pour la production de biopolymères exocellulaires à effet épaississant pour des milieux aqueux, par culture aérobie de souches de micro-organismes produisant les biopolymères, dans un milieu nutritif aqueux qui se trouve sous forme de phase aqueuse dispersée dans une émulsion eau-dans-huile (E/H) stable dans les conditions de fermentation, avec utilisation simultanée d'émulsifiants E/H pour la formation et la stabilisation d'émulsions, caractérisé en ce que l'on utilise la phase aqueuse en quantités inférieures à 50 % en volume - par rapport au mélange total - et on opère en ce cas avec des dialcanolamides d'acides gras en tant qu'émulsifiants E/H.

**2.** Procédé selon la revendication 1, caractérisé en ce que l'on utilise des diéthanolamides d'acides gras dont les radicaux acide gras peuvent également comporter une ou plusieurs insaturations éthyléniques.

**3.** Procédé selon la revendication 1 ou 2, caractérisé en ce que l'on utilise l'émulsifiant en quantités de 0,5 à 2 % en poids, de préférence de 0,7 à 1,2 % en poids, dans chaque cas par rapport au mélange total.

**4.** Procédé selon l'une quelconque des revendications 1 à 3, caractérisé en ce que l'on opère avec des hydrocarbures isoparaffiniques liquides à la température du processus (30 ± 5°C) ou des triglycérides liquides, en particulier des huiles comestibles, en tant que phase homogène.

**5.** Procédé selon l'une des revendications 1 à 4, caractérisé en ce que l'on utilise la phase huileuse ou une quantité de 30 à 40 % en volume - par rapport au mélange total.

**6.** Procédé selon l'une quelconque des revendications 1 à 5, caractérisé en ce que l'on ajoute dans la phase aqueuse dispersée des rendements en biopolymères d'au moins 5 % en poids - par rapport au mélange total - avant d'envoyer le produit du processus à la séparation de biopolymères.

**7.** Procédé selon l'une quelconque des revendications 1 à 6, caractérisé en ce que, pour la production de biopolymères de type polysaccharide, on utilise en particulier le genre Xanthomonas.